# FASCICULE DE BREVET EUROPEEN

(11) **EP 1 083 842 B1**
(45) Date de publication et mention de la délivrance du brevet: **07.07.2004**
(21) Numéro de dépôt: 00915288.5
(22) Date de dépôt: 05.04.2000
(51) Int. Cl.: A61F 2/00, A61B 17/064

(54) **PROTHESE SOUPLE NOTAMMENT POUR LA CURE DES HERNIES PAR VOIE COELIOSCOPIQUE**
WEICHPROTHESE, INSBESONDERE ZUR HEILUNG VON HERNIA MITTELS LAPAROSKOPIE
FLEXIBLE PROSTHESIS IN PARTICULAR FOR CURING HERNIAS BY THE CELIOSCOPIC PATH

(30) Priorité: 08.04.1999 FR 9904390
(43) Date de publication de la demande: 21.03.2001
(73) Titulaire: Johnson & Johnson International, New Brunswick, NJ 08933-7003 (US)
(72) Inventeur: LECALVE, Jean-Luc, F-35000 Rennes (FR)
(74) Mandataire: Texier, Christian
(86) Numéro de dépôt international: PCT/FR2000/000845
(87) Numéro de publication internationale: WO 2000/061033

(56) Documents cités:
- DE-A- 19 711 288
- FR-A- 2 710 518
- US-A- 3 810 467
- US-A- 5 333 624
- US-A- 5 810 851

## Description

La présente invention concerne le domaine des prothèses en tissu souple.

Elle s'applique en particulier aux prothèses destinées à être mises en place par chirurgie endoscopique.

La présente invention s'applique plus précisément encore de préférence à la fixation des prothèses souples définies dans les documents FR-A-2 710 518 et US-A-5 333 624. Les moyens définis dans ce document sont destinés principalement à la cure des hernies par voie coelioscopique à l'aide d'un treillis prédécoupé mis en place par voie laparoscopique.

Généralement les prothèses en tissu souple sont fixées par agrafage ou par suture.

L'agrafage est assez facile à réaliser pour la plupart des interventions à ciel ouvert. Il est plus délicat à opérer pour des interventions par coelioscopie. Dans tous les cas il reste assez onéreux.

La fixation de prothèses souples par suture est plus économique, mais elle est plus difficile à réaliser, notamment pour des interventions de type coelioscopie.

Dans le cadre du dispositif décrit dans le document précité FR-A-2 710 518 par exemple il est recommandé de réaliser la péritonisation avec une aiguille de fil résorbable de 10 à 15 cm de long bloqué tous les 3 ou 4 points par une pince.

On a certes envisagé d'éliminer les inconvénients de la technique antérieure en ayant recours à des colles biologiques. Cependant à la connaissance de la Demanderesse les tentatives dans ce sens n'ont pas donné satisfaction jusqu'ici.

La présente invention a ainsi pour but de proposer un nouveau dispositif qui permette d'éliminer les inconvénients de la technique antérieure en facilitant la fixation de prothèses souples sur un organe de corps vivant.

Ce but est atteint dans le cadre de la présente invention grâce à une prothèse souple comprenant au moins un dispositif d'ancrage (20) réalisé en un matériau à mémoire de forme et conçu pour être déformé par simple commande thermique, à partir d'une position de stockage dans laquelle le dispositif (20) est au moins sensiblement rectiligne et adjacent à la prothèse (10), dans une position de fixation dans laquelle ledit dispositif d'ancrage (20) a la forme générale d'une boucle largement en saillie par rapport à la prothèse (10) pour pénétrer dans les tissus avoisinants.

Selon une autre caractéristique avantageuse de la présente invention, ladite prothèse comporte plusieurs dispositifs d'ancrage parallèles entre eux en position initiale de stockage.

D'autres caractéristiques, buts et avantages de la présente invention apparaîtront à la lecture de la description détaillée qui va suivre et en regard des dessins annexés, donnés à titre d'exemples non limitatifs et sur lesquels :
- les figures 1 et 2 représentent deux vues schématiques de prothèses souples équipées de dispositifs d'ancrage conformes à la présente invention,
- la figure 3 représente une vue latérale d'un dispositif d'ancrage conforme à la présente invention, en position initiale de stockage, et
- les, figures 4 et 5 représentent respectivement le même dispositif d'ancrage, selon deux vues latérales orthogonales entre elles, en position de fixation.

La présente invention s'applique à tous types de prothèses souples.

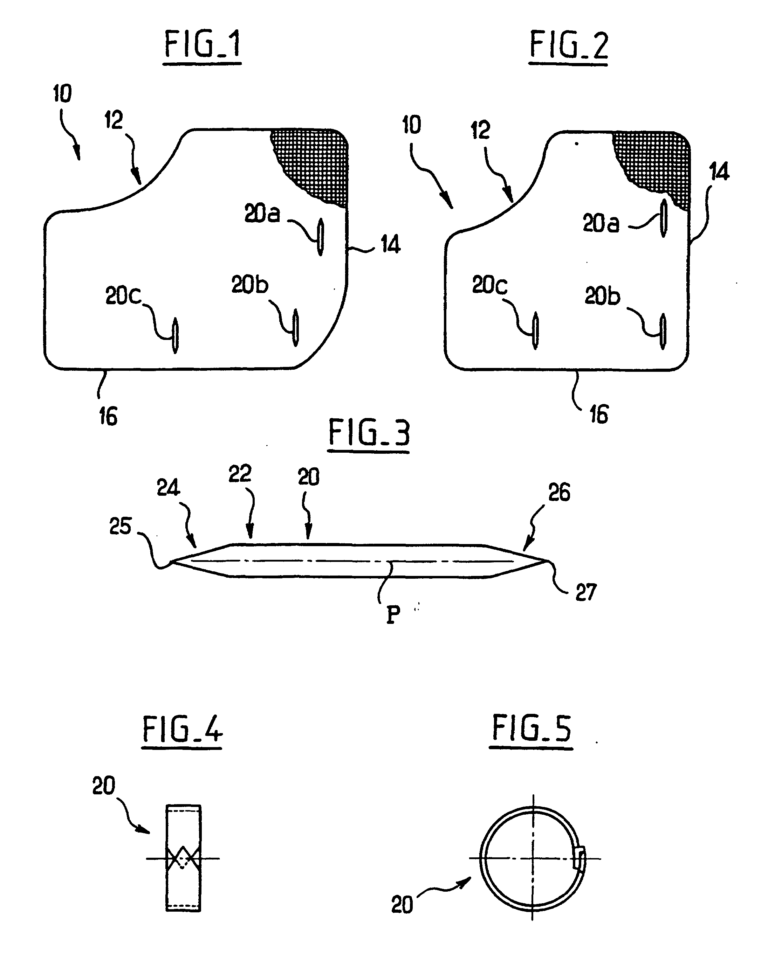

Elle s'applique cependant tout particulièrement aux prothèses 10 du type illustré sur les figures 1 et 2 annexées, conçues pour être mises en place avec un dispositif du type décrit dans le document FR-A-2 710 518.

De telles prothèses 10 destinées à la cure des hernies, ont un contour globalement rectangulaire à angles arrondis. Elles possèdent par ailleurs une découpe 12 dans leur angle inféro-externe épousant le relier du psoas.

Elles sont conditionnées à l'état plan dans un blister et associées à un moyen en forme de fourchette conçu pour enrouler lesdites prothèses au moment de leur utilisation. Une fois enroulée les prothèses sont déplacées sur leur site de pose à l'aide d'un trocart.

Comme on l'a indiqué précédemment, dans le cadre de la présente invention, la prothèse 10 est équipée d'au moins un dispositif d'ancrage 20 réalisé en un matériau à mémoire de forme et conçu pour être déformé par simple commande thermique, à partir d'une position de stockage, dans une position de fixation dans laquelle ledit dispositif d'ancrage 20 interfère avec les tissus environnants.

Plus précisément encore selon la présente invention, le dispositif d'ancrage 20 est de préférence conçu pour être déformé par simple commande thermique, à partir d'une position de stockage illustrée sur la figure 3 dans laquelle le dispositif 20 est au moins sensiblement rectiligne et adjacent à la prothèse 10, dans une position de fixation illustrée sur les figures 4 et 5 dans laquelle le dispositif 20 a la forme générale d'une boucle largement en saillie par rapport à la prothèse 10 pour pénétrer dans les tissus avoisinants.

Le dispositif d'ancrage 20 est de préférence réalisé en alliage TiNi à mémoire.

Il se présente de préférence au repos, avant déformation, sous forme d'un barreau rectiligne 22 à section droite rectangulaire dont les extrémités 24, 26 sont effilées en biseau.

Les extrémités 24, 26 en forme de dièdres définissent ainsi des pointes respectives 25, 27 situées dans le plan de symétrie longitudinale P du dispositif d'ancrage 20.

A titre d'exemple non limitatif :
la longueur totale du dispositif 20 est de l'ordre de 18 mm,
La largeur du dispositif 20 est de l'ordre de 1,62 mm,
L'épaisseur du dispositif 20 est de l'ordre de 0,25 mm et
L'angle des pointes d'extrémité du dispositif 20 est de l'ordre de 30°.

Par ailleurs le dispositif 20 est placé sur la prothèse dans une direction parallèle à l'axe d'enroulement de la prothèse 10.

Plus précisément encore dans le cadre de la présente invention, ladite prothèse 10 comporte de préférence plusieurs dispositifs d'ancrage 20 parallèles entre eux en position initiale de stockage. Ces différents dispositifs d'ancrage 20 sont par ailleurs également parallèles à l'axe d'enroulement de la prothèse 10.

Ainsi dans le cadre de la présente invention la prothèse 10 comporte de préférence trois dispositifs d'ancrage 20 disposés respectivement globalement dans les angles de la prothèse 10 autres que celui comportant la découpe 12.

Plus précisément encore comme on le voit sur les figures 1 et 2 annexées, de préférence deux des dispositifs d'ancrage, référencés 20a et 20b, ont leurs axes longitudinaux sensiblement alignés parallèles à un bord 14 de la prothèse 10, tandis que les dispositifs d'ancrage 20b et 20c parallèles entre eux ont leurs axes longitudinaux perpendiculaires à un autre bord 16 de la prothèse 10, qui est perpendiculaire au bord précité 14, ces deux derniers dispositifs d'ancrage 20b et 20c étant situés sensiblement à la même distance de cet autre bord 16.

Le dispositif d'ancrage 20 est initialement maintenu sur la prothèse 10 par simple entrelacement du dispositif 20 dans des mailles du treillis composant la prothèse 10.

Pour utiliser une prothèse conforme à la présente invention, il suffit lorsque cette prothèse 10 est acheminée, déployée et positionnée sur son site d'utilisation, de chauffer les dispositifs d'ancrage 20 par tous moyens appropriés, par exemple à l'aide d'un bistouri thermique classique, pour déformer les dispositifs d'ancrage vers leur position de fixation illustrée sur les figures 4 et 5, dans laquelle ces dispositifs pénètrent dans les tissus environnants.

Dans cette position de fixation les dispositifs d'ancrage 20 ont la forme d'une boucle circulaire fermée d'un diamètre moyen de l'ordre de 5 mm.

La Demanderesse a déterminé que la forme acérée des extrémités du dispositif d'ancrage 20 permettait une implantation satisfaisante de celui-ci dans les tissus, sans nécessiter de contre appui ou enclume comme cela est requis pour des agrafes classiques. La géométrie des dispositifs d'ancrage 20 conformes à la présente invention permet en effet une déformation progressive d'un état rectiligne d'origine à un état curviligne, en position de fixation, avec pénétration progressive, par ses extrémités, des dispositifs d'ancrage dans les tissus adjacents.

La prothèse 10 doit être réalisée en un matériau insensible à la température à laquelle réagit le matériau à mémoire de forme composant les dispositifs d'ancrage 20. Dans ce contexte de préférence la prothèse 10 est formée d'un tricot à base d'un monofilament synthétique non résorbable constitué d'un stéréo-isomère isotactique de polypropylène, par exemple de formule (C₃H₆)n.

La présente invention permet ainsi une fixation de la prothèse 10 beaucoup plus facile que tous les moyens de fixation jusqu'ici proposés.

## Revendications

1. Prothèse souple, notamment pour la cure des hernies par voie coelioscopique comprenant au moins un dispositif d'ancrage, **caractérisée par le fait que** ledit dispositif d'ancrage (20) est réalisé en un matériau à mémoire de forme et est conçu pour être déformé par simple commande thermique, à partir d'une position de stockage dans laquelle le dispositif (20) est au moins sensiblement rectiligne et adjacent à la prothèse (10), dans une position de fixation dans laquelle ledit dispositif d'ancrage (20) a la forme générale d'une boucle largement en saillie par rapport à la prothèse (10) pour pénétrer dans les tissus avoisinants.

2. Prothèse selon la revendication 1, **caractérisée par le fait qu'**elle comporte plusieurs dispositifs d'ancrage (20) parallèles entre eux en position initiale de stockage.

3. Prothèse selon l'une des revendications 1 ou 2, **caractérisée par le fait que** le dispositif d'ancrage (20) est réalisé en alliage TiNi à mémoire.

4. Prothèse selon l'une des revendications 1 à 3, **caractérisée par le fait que** le dispositif d'ancrage (20) se présente au repos, avant déformation, sous forme d'un barreau rectiligne (22) à section droite rectangulaire dont les extrémités (24, 26) sont effilées en biseau.

5. Prothèse selon la revendication 4, **caractérisée par le fait que** les extrémités (24, 26) du dispositif d'ancrage (20) sont en forme de dièdres et définissent des pointes respectives (25, 27) situées dans un plan de symétrie longitudinale (P) du dispositif d'ancrage (20).

6. Prothèse selon l'une des revendications 1 à 5, **caractérisée par le fait que** la longueur totale du dispositif d'ancrage (20) est de l'ordre de 18 mm.

7. Prothèse selon l'une des revendications 1 à 6, **caractérisée par le fait que** la largeur du dispositif d'ancrage (20) est de l'ordre de 1,62 mm.

8. Prothèse selon l'une des revendications 1 à 7, **caractérisée par le fait que** l'épaisseur du dispositif d'ancrage (20) est de l'ordre de 0,25 mm.

9. Prothèse selon l'une des revendications 1 à 8, **caractérisée par le fait que** l'angle des pointes d'extrémité (24, 26) du dispositif d'ancrage (20) est de l'ordre de 30°.

10. Prothèse selon l'une des revendications 1 à 9, **caractérisée par le fait que** chaque dispositif d'ancrage (20) est placé sur la prothèse (10) dans une direction parallèle à un axe d'enroulement de la prothèse (10).

11. Prothèse selon l'une des revendications 1 à 10, **caractérisée par le fait qu'**elle comporte trois dispositifs d'ancrage (20).

12. Prothèse selon l'une des revendications 1 à 11, **caractérisée par le fait qu'**elle comporte trois dispositifs d'ancrage (20) disposés respectivement à proximité de trois angles de la prothèse (10).

13. Prothèse selon l'une des revendications 1 à 12, **caractérisée par le fait qu'**elle possède un contour globalement rectangulaire à angles arrondis et possède par ailleurs une découpe (12) dans son angle inféro-externe épousant le relier du psoas.

14. Prothèse selon la revendication 13, **caractérisée par le fait qu'**elle comporte trois dispositifs d'ancrage (20) disposés globalement dans les angles de la prothèse (10) autres que celui comportant la découpe (12).

15. Prothèse selon l'une des revendications 11 à 14, **caractérisée par le fait qu'**elle comporte deux dispositifs d'ancrage (20a, 20b) dont les axes longitudinaux sont sensiblement alignés parallèles à un bord (14) de la prothèse (10), et deux dispositifs d'ancrage (20b, 20c) parallèles entre eux dont les axes longitudinaux perpendiculaires à un autre bord (16) de la prothèse (10) sont situés sensiblement à la même distance de cet autre bord (16).

16. Prothèse selon l'une des revendications 1 à 15, **caractérisée par le fait que** chaque dispositif d'ancrage (20) est initialement maintenu sur la prothèse (10) par entrelacement dans des mailles du treillis composant la prothèse (10).

17. Prothèse selon l'une des revendications 1 à 16, **caractérisée par** le fait dans la position de fixation les dispositifs d'ancrage (20) ont la forme d'une boucle circulaire fermée.

18. Prothèse selon l'une des revendications 1 à 17, **caractérisée par** le fait dans la position de fixation les dispositifs d'ancrage (20) ont la forme d'une boucle circulaire fermée d'un diamètre moyen de l'ordre de 5 mm.

19. Prothèse selon l'une des revendications 1 à 18, **caractérisée par le fait qu'**elle est réalisée en un matériau insensible à la température à laquelle réagit le matériau à mémoire de forme composant les dispositifs d'ancrage (20).

20. Prothèse selon l'une des revendications 1 à 19, **caractérisée par le fait qu'**elle est formée d'un tricot à base d'un monofilament synthétique non résorbable constitué d'un stéréo-isomère isotactique de polypropylène, par exemple de formule (C₃H₆)ₙ.

## Patentansprüche

1. Biegsame Prothese, insbesondere zur Behandlung von Hemien auf laparoskopischem Weg, die wenigstens eine Verankerungsvorrichtung umfaßt,
**dadurch gekennzeichnet, daß** die Verankerungsvorrichtung (20) aus einem Material mit Formgedächtnis gefertigt und dafür vorgesehen ist, durch einfache Wärmesteuerung, ausgehend von einer Lagerungsstellung, in der die Vorrichtung (20) wenigstens in etwa gerade und benachbart zur Prothese (10) ist, in eine Befestigungsstellung verformt zu werden, in der die Verankerungsvorrichtung (20) im wesentlichen die Form einer Schleife hat, die weit aus der Prothese (10) vorsteht, um in die benachbarten Gewebe einzudringen.

2. Prothese nach Anspruch 1, **dadurch gekennzeichnet, daß** sie mehrere Verankerungsvorrichtungen (20) umfaßt, die in ihrer anfänglichen Lagerungsstellung untereinander parallel sind.

3. Prothese nach einem der Ansprüche 1 oder 2, **dadurch gekennzeichnet, daß** die Verankerungsvorrichtung (20) aus einer TiNi-Legierung mit Gedächtniseffekt gefertigt sind.

4. Prothese nach einem der Ansprüche 1 bis 3, **dadurch gekennzeichnet, daß** die Verankerungsvorrichtung (20) in der Ruhestellung vor der Verformung die Form eines geradlinigen Stabs (22) mit rechteckigem Querschnitt aufweist, dessen Enden (24, 26) in abgeschrägten Kanten spitz zulaufen.

5. Prothese nach Anspruch 4, **dadurch gekennzeichnet, daß** die Enden (24, 26) der Verankerungsvorrichtung (20) V-förmig sind und zugehörige Spitzen (25, 27) ausbilden, die sich in einer in Längsrichtung verlaufenden Symmetrieebene (P) der Verankerungsvorrichtung (20) befinden.

6. Prothese nach einem der Ansprüche 1 bis 5, **dadurch gekennzeichnet, daß** die Gesamtlänge der Verankerungsvorrichtung (20) in der Größenordnung von 18 mm ist.

7. Prothese nach einem der Ansprüche 1 bis 6, **dadurch gekennzeichnet, daß** die Breite der Verankerungsvorrichtung (20) in der Größenordnung von 1,62 mm ist.

8. Prothese nach einem der Ansprüche 1 bis 7, **dadurch gekennzeichnet, daß** die Dicke der Verankerungsvorrichtung (20) in der Größenordnung von 0,25 mm ist.

9. Prothese nach einem der Ansprüche 1 bis 8, **dadurch gekennzeichnet, daß** der Winkel der Endspitzen (24, 26) der Verankerungsvorrichtung (20) in der Größenordnung von 30° ist.

10. Prothese nach einem der Ansprüche 1 bis 9, **dadurch gekennzeichnet, daß** jede Verankerungsvorrichtung (20) auf der Prothese (10) in einer Richtung angeordnet ist, die parallel zu einer Aufrollachse der Prothese (10) ist.

11. Prothese nach einem der Ansprüche 1 bis 10, **dadurch gekennzeichnet, daß** sie drei Verankerungsvorrichtungen (20) umfaßt.

12. Prothese nach einem der Ansprüche 1 bis 11, **dadurch gekennzeichnet, daß** sie drei Verankerungsvorrichtungen (20) umfaßt, die jeweils in der Nähe dreier Ecken der Prothese (10) angeordnet sind.

13. Prothese nach einem der Ansprüche 1 bis 12, **dadurch gekennzeichnet, daß** sie eine im wesentlichen rechteckige Kontur mit abgerundeten Ecken aufweist und außerdem in ihrer unteren-äußeren Ecke einen Ausschnitt (12) aufweist, der sich der Form des Lendenmuskels anpaßt.

14. Prothese nach Anspruch 13, **dadurch gekennzeichnet, daß** sie drei Verankerungsvorrichtungen (20) umfaßt, die in etwa in den Ecken der Prothese (10) angeordnet sind, die nicht den Ausschnitt (12) aufweisen.

15. Prothese nach einem der Ansprüche 11 bis 14, **dadurch gekennzeichnet, daß** sie zwei Verankerungsvorrichtungen (20a, 20b), deren Längsachsen im wesentlichen parallel zu einem Rand (14) der Prothese (10) ausgerichtet sind, und zwei zueinander parallele Verankerungsvorrichtungen (20b, 20c), deren senkrecht zu einem anderen Rand (16) der Prothese (10) stehende Längsachsen im wesentlichen im selben Abstand von diesem anderen Rand (16) angeordnet sind, umfaßt.

16. Prothese nach einem der Ansprüche 1 bis 15, **dadurch gekennzeichnet, daß** jede Verankerungsvorrichtung (20) anfänglich durch Verschlingung mit Maschen des die Prothese (10) bildenden Geflechts auf der Prothese (10) gehalten wird.

17. Prothese nach einem der Ansprüche 1 bis 16, **dadurch gekennzeichnet, daß** die Verankerungsvorrichtungen (20) in der Befestigungsstellung die Form einer geschlossenen kreisförmigen Schleife haben.

18. Prothese nach einem der Ansprüche 1 bis 17, **dadurch gekennzeichnet, daß** die Verankerungsvorrichtungen (20) in der Befestigungsstellung die Form einer geschlossenen kreisförmigen Schleife mit einem mittleren Durchmesser in der Größenordnung von 5 mm haben.

19. Prothese nach einem der Ansprüche 1 bis 18, **dadurch gekennzeichnet, daß** sie aus einem Material gefertigt ist, das gegenüber der Temperatur, bei der das die Verankerungsvorrichtungen (20) bildende Material mit Formgedächtnis reagiert, unempfindlich ist.

20. Prothese nach einem der Ansprüche 1 bis 19, **dadurch gekennzeichnet, daß** sie aus einem Gewebe gebildet ist, das auf einem nicht-resorbierbaren synthetischen Einzelfaden basiert, der aus einem isotaktischen Polypropylen-Stereoisomer, beispielsweise mit der Formel (C₃H₆)ₙ, besteht.

## Claims

1. A flexible prosthesis, in particular for curing hernias by colioscopy the prosthesis including at least one anchor device, the prosthesis being **characterized by** the fact that said anchor device (20) is made of shape memory material and is designed to be deformed merely under temperature control from a storage position in which the device (20) is at least substantially rectilinear and adjacent to the prosthesis (10) to a fixing position in which said anchor device (20) is generally in the form of a loop projecting considerably from the prosthesis (10) so as to penetrate into the surrounding tissue.

2. A prosthesis according to claim 1, **characterized by** the fact that it includes a plurality of anchor devices (20) that are mutually parallel in the initial, storage position.

3. A prosthesis according to claim 1 or 2, **characterized by** the fact that the anchor device (20) is made of a memory TiNi alloy.

4. A prosthesis according to any one of claims 1 to 3, **characterized by** the fact that the anchor device (20) when at rest, prior to deformation, is in the form of a rectilinear strip (22) of rectangular right-section having ends (24, 26) which taper in chamfers.

5. A prosthesis according to claim 4, **characterized by** the fact that the ends (24, 26) of the anchor device (20) are V-shaped and define respective points (25, 27) situated in a longitudinal plane of symmetry (P) of the anchor device (20).

6. A prosthesis according to any one of claims 1 to 5, **characterized by** the fact that the total length of the anchor device (20) is about 18 mm.

7. A prosthesis according to any one of claims 1 to 6, **characterized by** the fact that the width of the anchor device (20) is about 1.62 mm.

8. A prosthesis according to any one of claims 1 to 7, **characterized by** the fact that the thickness of the anchor device (20) is about 0.25 mm.

9. A prosthesis according to any one of claims 1 to 8, **characterized by** the fact that the angle of the end points (24, 26) of the anchor device (20) is about 30°.

10. A prosthesis according to any one of claims 1 to 9, **characterized by** the fact that each anchor device (20) is placed on the prosthesis (10) in a direction that is parallel to the axis about which the prosthesis (10) is rolled up.

11. A prosthesis according to any one of claims 1 to 10, **characterized by** the fact that it has three anchor devices (20).

12. A prosthesis according to any one of claims 1 to 11, **characterized by** the fact that it has three anchor devices (20) disposed in the vicinity of three respective corners of the prosthesis (10).

13. A prosthesis according to anyone of claims 1 to 12, **characterized by** the fact that it possesses a generally rectangular outline having rounded corners and also possesses a cutout (12) in its infero-lateral corner that fits over the bulge of the psoas.

14. A prosthesis according to claim 13, **characterized by** the fact that it has three anchor devices (20) disposed generally in the corners of the prosthesis (10) other than its corner that includes the cutout (12).

15. A prosthesis according to any one of claims 11 to 14, **characterized by** the fact that it has two anchor devices (20a, 20b) whose longitudinal axes are substantially in alignment parallel to one of the edges (14) of the prosthesis (10), and two mutually parallel anchor devices (20b, 20c) whose longitudinal axes are perpendicular to another edge (16) of the prosthesis (10) and which are situated at substantially the same distance from the other edge (16).

16. A prosthesis according to any one of claims 1 to 15, **characterized by** the fact that each anchor device (20) is initially secured to the prosthesis (10) by being interlaced through the mesh of the web constituting the prosthesis (10).

17. A prosthesis according to any one of claims 1 to 16, **characterized by** the fact that the shape of the anchor devices (20) in the fixing position is that of a closed circular loop.

18. A prosthesis according to any one of claims 1 to 17, **characterized by** the fact that the shape of the anchor devices (20) in the fixing position is that of a closed circular loop having a mean diameter of about 5 mm.

19. A prosthesis according to anyone of claims 1 to 18, **characterized by** the fact that it is made of a material that is insensitive to the response temperature of the shape memory material constituting the anchor devices (20).

20. A prosthesis according to anyone of claims 1 to 19, **characterized by** the fact that it is constituted by a knit based on a non-resorbable synthetic monofilament constituted by an isotatic stereo-isomer of polypropylene, e.g. having the formula (C₃H₆)ₙ.
